# DEMANDE DE BREVET EUROPEEN

(11) **EP 3 889 607 A1**
(43) Date de publication de la demande: **06.10.2021**
(21) Numéro de dépôt: 21166711.8
(22) Date de dépôt: 01.04.2021
(51) Int. Cl.: G01N 33/558, G01N 33/569

(54) **MÉTHODE DE DÉPISTAGE IN VITRO CÔTÉ PATIENT POUR LE DIAGNOSTIC RAPIDE DU SARS-COV-2**

(30) Priorité: 02.04.2020 MC 2698
(71) Demandeur: Moffa, Ricardo, 98000 Monaco (MC); De Rosa, Alfredo, 80131 Napoli (IT); Di Domenico, Marina, 80133 Napoli (NA) (IT); Boccellino, Mariarosaria, 80127 Napoli (NA) (IT)
(72) Inventeur: Moffa, Ricardo, 98000 Monaco (MC); De Rosa, Alfredo, 80131 Napoli (IT); Di Domenico, Marina, 80133 Napoli (NA) (IT); Boccellino, Mariarosaria, 80127 Napoli (NA) (IT)
(74) Mandataire: Mola, Edoardo

(57) **Abrégé**

Méthode de dépistage in vitro coté patient pour le diagnostic rapide du SARS-COV-2. Cette invention fait partie du diagnostic rapide des stades symptomatiques ou asymptomatiques des infections virales dans l'homme, en particulier celui du SARS-CoV-2 (coronavirus du syndrome respiratoire aigu 2) à l'aide d'un kit, stable à température ambiante, base sur des méthodes immunologiques pour la recherche de protéines virales spécifiques sur les sécrétions prélevés avec l'écouvillon de la cavité oro rhinopharyngée.

Le kit a un deuxième domaine industriel et, avec un outil d'échantillonnage approprié représenté par des tampons plus grands et humidifiés et o une serviette, sera utilisé pour vérifier si dans les conduits et les filtre de climatisation ou aération des : bâtiments, locales notamment publique, moyens de transport public inclusif le navire, si il y a une trace de contamination du SARS COV-2, cette application industrielle est également nécessaire parce qu'elle est bien établie et prouvée par des études que le virus persiste et se propage à travers les systèmes de ventilation de la climatisation.

## Description

### DOMAINE TECHNIQUE

Cette invention fait partie du diagnostic rapide des stades symptomatiques ou asymptomatiques des infections virales, en particulier celui du SARS-CoV-2 (coronavirusgravegrave du syndrome respiratoire aigu 2) à l'aide d'un kit, stable à température ambiante, basé sur des méthodes immunologiques pour la recherche de protéines virales spécifiques sur les Sécrétions prélevés avec l'écouvillon de la cavité bucco rhinopharyngée. La trousse d'invention permet de diagnostiquer de manière non invasive et fiable l'existence d'un état infectieux (pathologie connue sous le nom de COVID 19, acronyme pour CO-rona VI-rus D-isease année d'identification, 2019), également tôt, et de formuler rapidement l'état de contagion et l'adoption de protocoles d'isolement / quarantaine dans les deux stratégies thérapeutiques symptomatiques et asymptomatiques. Le kit a un deuxième domaine industriel et, avec un outil d'échantillonnage approprié représenté par des tampons plus grands et humidifiés et o une serviette, sera utilisé pour vérifier si dans les conduits et les filtre de climatisation où aération des: bâtiments, locales notamment publique, moyens de transport public inclusif le navire, il y a une trace de contamination du SARS COV-2, cette application industrielle est également nécessaire parce qu'elle est bien établie et prouvée par des études que le virus persiste et se propage à travers les systèmes de ventilation de la climatisation.

### Etat de la technique antérieure

Dans la pandémie grave actuelle du SARS-CoV-2, il est nécessaire d'identifier des dispositifs pour contrôler la propagation de l'infection virale. Au moment de cette invention, la pandémie ne peut être combattue par des médicaments déclares spécifiques ou empêchés par l'utilisation d'un vaccin, laissant la distance sociale et la quarantaine de la population asymptomatique infectée ou positive à la distance sociale et à la quarantaine de la population. Mieux contraster avec ce virus. Dans le contexte des manifestations symptomatiques et asymptomatiques du district tête-cou, il y a une concentration de virus dans les narines, le pharyngé et dans une moindre mesure dans la cavité buccale.

Le dépistage rapide devient donc d'une importance primordiale pour orienter le médecin vers le plan de traitement et surtout pour adopter dès que possible l'isolement des sujets symptomatiques et asymptomatiques de sujets sains, ou de la même manière éviter quarante inutile et nocif d'un point de vue socio-économique des patients présentant des symptômes DE COVID19, mais sans le virus du SARS-CoV-2. À l'heure actuelle, la méthode de dépistage scientifiquement fiable est l'essai de l'écouvillon or rhinopharyngé pour l'analyse de l'ARN en laboratoire, ce qui implique des dépenses économiques importantes et du temps long pour le résultat. Ces retards réduisent considérablement I efficacité des protocoles de quarantaine, mettant les gens près de la personne en attente d'un résultat en danger. Le cout élevé limite également l'utilisation des essais à grande échelle. Il convient de noter que dans l'étude épidémiologique réalisée dans Codogno 50% des points positifs étaient asymptomatiques et donc des sujets qui propagent la contagion parce qu'ils n'ont pas été vérifiés pour leur positivité au virus.

Une autre méthode de screening sont des tests rapides, souvent dans la technique de flux latéral, qui utilisent le sang ou le sérum pour rechercher des anticorps spécifiques tels que l'IgM et l'IgG développes par des patients qui ont contracte le virus SARS-CoV-2. Des études scientifiques ont montré une faible efficacité de ces tests (déclares 80% de faux positifs) à un point tel que la CTS italienne a dû brancher des circulaires aux hôpitaux et aux ASL pour instruire de ne pas les utiliser, même comme premier dépistage. Il est donc nécessaire de fournir une méthode pour un diagnostic de l'infection par le SARS-CoV-2 à caractère qualitatif, rapide, sensible et spécifique qui est basée sur la reconnaissance d'antigènes spécifiques au virus SARS-CoV-2, une méthode qui peut également être effectuée dans des environnements à l'extérieur du laboratoire, « côté patient » avec réponse immédiate du caractère chimio colorimétrique Ou lecteur numérique. En ce qui concerne le deuxième domaine industriel, il n'existe actuellement aucun système de dépistage ou de kits portatifs du SARS COV 2 pour vérifier rapidement que les surfaces sont contaminées par le SARS-CoV-2.

### DESCRIPTION DE L'INVENTION

Ce brevet revendique un kit innovant avec la technique ELISA et un résultat chimio colorimétrique ou numérique pour la détection précoce de l'infection par le SARS-CoV-2 aux stades symptomatiques ou asymptomatiques. Le kit utilise des réactifs stables à température ambiante, il est rapide, sensible, spécifique, transportable, bon marché et non-invasif. En particulier, le kit d'invention permet de déterminer deux PROTEINS de SARS-CoV-2, de protéines S et de protéines N (Nucléocapside) par des anticorps primaires à double utilisation pour assurer sans équivoque la spécificité et sensibilité du signal. En particulier, un premier ensemble d'anticorps primaire anti-SARS Virus COV-2 Spike Protéine et anti-SARS Virus COV-2 Protéine nucléoside, sera adhésives sur une membrane de PVDF, un deuxième ensemble d'anticorps primaires d'une espèce diffèrent anti-SARS Virus COV-2 Spike Protéine et anti-SARS Virus COV-2 Nucléocapside Protéine sera utilisé dans les tubes à essai. Des anticorps secondaires anti-espèce de le deuxième anticorps primaire dans la station numéro 2MA conjugués à un système d'amélioration du signal enzymatique en particulier le phosphate ou la peroxydase seront utilisés.

Le kit utilise des anticorps sèches ou lyophilises, stables à température ambiante, qui au moment de l'utilisation sont solubles dans une solution tampon présente dans le kit.

Cette invention est une aide diagnostique efficace pour l'infection par le SARS-CoV-2, qui ne nécessite pas d'aides de laboratoire et fournit des réponses immédiates.

L'invention fait également référence à la création d'un kit innovant construit en assemblant des produits à moitié finis disponibles sur le marché et sur mesure. Le kit comprend un outil (Cytobrush et/ou l'écouvillon nasal et/ou l'écouvillonnement de balises) utile pour la collecte de l'échantillon biologique au niveau de l'oro rhinopharynx muqueuse et le nécessaire pour permettre au professionnel (médecin ou personne titrée pour une utilisation par la réglementation actuelle dans le territoire d'utilisation) de l'analyser et de vérifier la présence de biomarqueurs viraux ou de le traiter par des dispositifs de développement automatique spécifiques. Le produit a une façon extrêmement simple de l'utiliser.

Cette invention est décrite ci-dessous dans des exemples non limitatifs, se référant aux chiffres suivants:
Composants : kit équipé d'un outil tampon pour la collecte de matériel biologique (bâton équipé de cytobrush ou de coton) et, PVDF (polyvinilidenfluoride) (polyvinilidenfluoride) monté de façon appropriée sur support rigide et tubes à essai préchargés de poudres d'inhibiteurs de la protéase à l'état sec, lyophilisés et/ou des anticorps séchés, tubes préchargés avec des liquides, liquides de lavage, développement et détection du signal et des dispositifs enzymatiques pour reporter en état soluble les réactifs préchargés, en particulier des bouchons à double face.

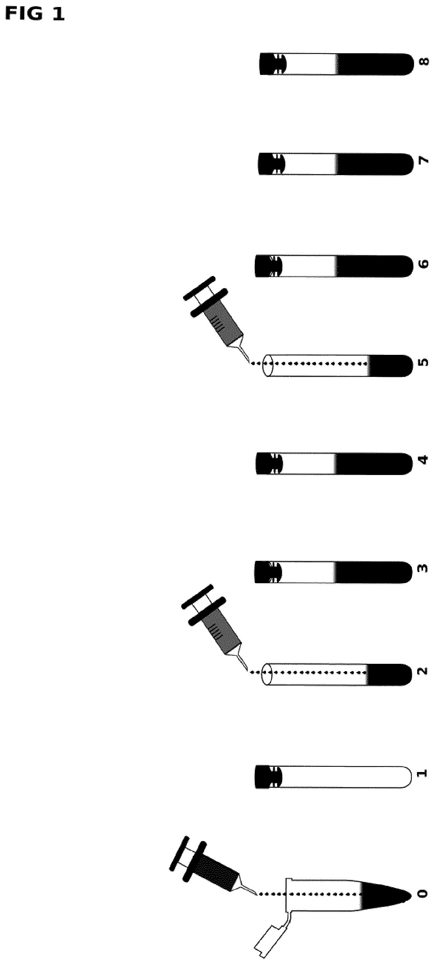

Figure 1. Schéma d'exploitation du kit : (2a) ramener à la solution les réactifs qui se trouvent clans les puits/station clans un état de séchage et/ou lyophilisés à l'ouverture du kit enlever les cellules de la muqueuse d'oro rhinopharyngée et excrétés potentiellement infectés par les outils spécifiques pour faire le tampon; plongée de la même dans le puits/station zéro, bien contenant un inhibiteur de la protéase et un tampon lisant les cellules potentiellement infectées et excrétés prélevée par le dispositif de collecte.

Schéma de transfert de la lyse du poste de pilotage/station zéro au poste de pilotage/station un ; immersion de la bande PVDF dans le cockpit/station un pour la reconnaissance des antigènes viraux par des anticorps primaires immobilises sur la bande PVDF. Dans le poste de pilotage/station, on se produit la formation d'immunocomplexes sur la bande s'il y a présence de protéines virales dans l'échantillon prélevé.

Système de plongée à bande PVDF clans le cockpit / station numéro deux. La bande PVDF avec immunocomplexes immobilisés est immergé clans le cockpit / station numéro deux contenant une solution d'anticorps primaires tout aussi spécifiques aux antigènes viraux, mais d'une espèce autre que celles adsorbent dans membrane. L'utilisation de cette deuxième série d'anticorps primaires est destinée à permettre une reconnaissance spécifique par l'anticorps secondaire utilisé dans la phase suivante, empêchant ce dernier d'être en mesure de se lier à tous les anticorps primaires adsorbé sur la bande PVDF. Dans les puits/stations numéro trois et quatre, des lavages T-PBS auront lieu.

Schéma d'immersion dans le poste de pilotage/station numéro cinq de la bande PVDF sur laquelle des immunocomplexes primaires d'antigène/anticorps sont présents pour permettre l'interaction avec des anticorps secondaires conjugués à un système d'enzymes de détection ; dans les puits/stations numéro six et sept seront lavés avec T-PBS ; Expression de couleur utilisant le substrat stable à température ambiante dans le poste de pilotage/station numéro huit.
Lecture et interprétation du résultat.

### EXAMPLE

### EXPLORE 1 - Les marqueurs biologiques Protéines Spike et Nucléocapside.

La méthode pour le diagnostic et la surveillance précoces de la maladie de SARS-CoV-2 dans ce brevet implique la détection de deux antigènes viraux (protéine de pointe et nucléocapside) dans des échantillons prélevés dans le district oro rhinopharyngé du sujet à l'examen. Les marqueurs "S-protéine (Spike protéine) et N-protéine (Nucléocapside)" dans le cadre de cette invention comprennent également des fragments, des dérivés, des variantes, des isoformes, etc. Ces marqueurs sont caractérisés par les numéros d'accès NCBI et les anticorps utilisés pour la préparation de l'appareil se réfèrent de préférence à ce qui suit pris comme exemple : SARS-CoV/SARS-CoV-2 (COVID-19) anticorps à pointe [1A9] GTX632604 (monoclonal de souris) ; Anticorps nucléosides SARS- CoV/SARS-CoV-2 (COVID-19) [6H3] Cat No. GTX632269 (souris monoclonale) ; Souris IgG (Fc fragment) anticorps, F(ab') 2 fragments, pré-adsorbé (AP) Cat No. GTX25880 (Chèvre, Polyclonal) ; Rabbit anti-SARS Virus Spike Protéine Code : RB128-10168-2 ; Rabbit anti-SARS Virus Code des protéines nucléosides RB128-10165-2; 2019-nCoV Spike Protéine (SI -S2 ECD, His tag) Code SI40589-V08B1-100; 2019-nCoV Nucléocapside Protéine (His tag) Code SI40588-V08B-100

### Protocole de préparation de bande

### EXAMPLE 2 - PVDF

Les étapes de préparation de la bande PVDF armées des principaux anticorps d'intérêt dans la séquence sont les suivantes: l'hydratation de la bande PVDF (ThermoFisher Scientific Catalogue Number LC2002) avec du méthanol pendant 5 min (Fig.8, Panneau A); laver avec de l'eau pendant 5 minutes; deux lavages avec PBS pendant 5 min; incubation avec protéine A-biotine de *Staphylococcus aureus* (Sigma-Aldrich, 10g/mL) par 1 h dans PBS; deux lavages avec PBS (; verrouillage avec 3% de solution BSA dans PBS pour 1 h; trois lavages avec PBS pendant 5 min; assemblage PVDF incubation avec solution d'anticorps de lapin (Rabbit anti-SARS Virus Spike Protéine code: RB 128-10168-2; Rabbit anti-SARS Virus Code des protéines nucléoïdes RB128-10165-2) environ 400 L par canal, O.N. ; trois lavages avec PBS pendant 5 min ; deux lavages de filtre avec PBS TEA 0.2M ;DMP 25mM lave dans TEA HC1 0.2M pH 8.2 , incubation avec TEA 0.2 M - 20 mM éthanolamine ; deux lavages avec PBS pendant 5 min ; stockage en 0,02 NaN₃ dans PBS ; le filtre est coupé perpendiculaire aux canaux dans lesquels les anticorps primaires anti-SARS Virus Spike Protéine et anti-SARS Virus Nucléocapside Protéine (pour la capture de ses antigènes libérés par l'échantillon recueilli) ont été chargés pour obtenir une bande de largeur égale à environ 0,4 cm, qui sera monté sur le support de l'échantillon. La bande est ensuite traitée avec la méthode ELISA afin de mettre en évidence les antigènes spécifiques présents dans l'échantillon cytologique testé.

Le contrôle positif (CTR) est un anticorps anti-actine (Sigma-Aldrich, A2066).

### EXAMPLE 3 - Description de l'appareil

Voici les différents composants du kit équipés de tampon pour la collecte de matériel biologique et le soutien de la bande PVDF (Immobilon) armé d'anticorps primaires d'intérêt. La boîte de kit est organisée en rangées de neuf puits/stations contenant des tampons, des systèmes de lecture, des systèmes de révélation stables à température ambiante et des anticorps secs/gelés stables à température ambiante.

### EXAMPLE 4 - Comment utiliser le kit

Voici les étapes opérationnelles qui permettent le diagnostic de l'infection virale : prélever au moins deux échantillons, l'un dans la région oropharyngé et l'autre dans la région de rhinopharyngé, à l'aide des outils de collecte présents dans le kit ; plonger le tampon avec l'échantillon biologique pendant 15 min dans le puits/station numéro zéro contenant le tampon lysée; l'extraction de l'instrument et le transfert de la lessive dans le cockpit/station numéro un ; l'ouverture du paquet de soutien auquel la membrane PVDF est adhésive; le support est plongé avec la bande PVDF pendant 5 minutes dans le premier cockpit/station numéro un pour permettre l'interaction des protéines (antigènes) avec les anticorps primaires adhésifs à la bande PVDF, afin de former immunocomplexe (à titre d'exemple: anticorps à pointes SARS-CoV-2 [IA9] Cat No. GTX632604 (souris monoclonale); SARS-CoV/SARS-CoV-2 (anticorps nucléocapside [6H3] Cat No. GTX632269 (souris monoclonale) ; Souris IgG (Fc fragment) anticorps, F(ab') 2 fragments, pré-adsorbé (AP) Cat No. GTX25880 (Chèvre, Polyclonal); Rabbit anti-SARS Virus Spike Protéine Code: RB128-10168-2; Rabbit anti-SARS Virus Code des protéines nucléopsides RB128-10165-2; 2019-nCo V Spike Protéine (S1-S2 ECD, His tag) Code SI40589-V08B 1-100;2019-nCoV Nucléocapside Protéine (His tag) Code SI40588-V08B-100; ensuite, la membrane PVDF plonge dans le cockpit / station numéro deux contenant en solution la deuxième série d'anticorps de souris primaires SARS-CoV/SARS-CoV-2 (COVID-19) anticorps Spike et SARS-Co V/SARS-Co V-2 (COVID-19) noyau d'anticorps pour la réaction avec l'immunocomplexe immobilisé; si procède avec 2 lavages de soutien avec la bande PVDF dans les puits contenant T-PBS-buffer pour éliminer les protéines aspécifiques adhéré à l'immunocomplexe ; le stationnement de support avec la bande PVDF pendant 10 min dans le puits / station numéro cinq contenant l'anticorps secondaire de la souris équipé d'un système d'enzymes révélatrices (phosphate d'alcaline ou peroxydase,); 2 lavages de soutien avec bande PVDF dans les puits/station numéro six et sept contenant T-PBS-buffer pour éliminer l'excès d'anticorps secondaires ; tremper le support avec la bande PVDF dans le bien / station numéro huit contenant le substrat (BCIP /NBT, stable à température ambiante) nécessaire pour la réaction de couleur ; interprétation du résultat.

### EXAMPLE 5 - Amplifier le signal en fonction de la température.

L'affichage colorimétrique sera effectué à température ambiante, correspondant à une plage de température comprise entre 20 et 30 degrés Celsius. L'utilisation, dans le kit, d'un anticorps secondaire équipé d'un système d'enzymes révélatrices (phosphate d'alcaline ou peroxydas) trouve dans cette plage de température, l'activité enzymatique maximale, permettant ainsi une amplification optimale du signal.

### EXAMPLE 6 - Utilisation de la trousse d'invention pour identifier les personnes infectées par le SARS-CoV-2

Le kit a été utilisé en supposant que chez ceux qui ont donné un résultat positif à l'infection par le SARS-CoV-2, il y avait une présence minimale de 1 nanogramme d'antigène viral dans 1 microlitres de la solution Lissée, et des tests de concentration ont ensuite été effectués pour voir la capacité de capture des signal (valeur de cut off of the signal) du système Object de ce brevet. Les résultats expérimentaux démontrent la pleine conformité aux quantité retrouvable sur la muqueuse des patients symptomatiques et asymptomatiques, et donc valide l'efficacité de ce system et méthode de ce brevet.

### Légende figure 1

- Image 0 : station numéro zéro du kit covid
- Image 1 : station numéro un du kit covid
- Image 2 : station numéro deux du kit covid
- Image 3 : station numéro trois du kit covid
- Image 4 : station numéro quatre du kit covid
- Image 5 : station numéro cinq du kit covid
- Image 6 : station numéro six du kit covid
- Image 7 : station numéro sept du kit covid
- Image 8 : station numéro huit du kit covid

## Revendications

1. *Méthode in vitro* pour identifier la positivité à l'INFECTION avec le SARS-CoV-2 et les mutations subséquentes chez l'homme à l'aide d'un kit qui permet d'effectuer un test latéral patient ELISA, avec des réactifs séchés ou lyophilisés, qui sont remis en solution et/ou mélangés au moment de l'utilisation du kit, obtenant une réponse qualitative immédiate chimio colorimétrique ou numérique, y compris les étapes suivantes : a) détectent et/ou quantifient au moins l'un des deux antigènes spécifiques au virus SARS-CoV-2 (protéine Spike et/ou quantifier Nucléocapside) dans un échantillon biologique obtenu à partir du sujet; b) comparer le résultat avec un échantillon témoin; La protéine Spike et/ou la nucléocapside indiquent que le sujet est infecté par le SARS-CoV-2.

2. Méthode selon la revendication 1 qui implique l'utilisation d'un kit contenant des composants qui permettent les activités suivantes: prise de l'échantillon biologique dans la région bucco rhinopharyngé par tampon; immobilisation sur une membrane PVDF des anticorps primaires ant-SARS-Co V-2 Spike Protéine et ant-SARS-Co V-2 Nucléocapside Protéine; formation du complexe primaire d'antigène/anticorps en reliant l'échantillon biologique avec la membrane PVDF portant des anticorps primaires immobilisé; formation complexe entre le système primaire d'antigène/anticorps immobilisé sur PVDF et une deuxième série d'anticorps primaires ant-SARS-CoV-2 Spike Protéine et ant-SARS-Co V-2 Nucléocapside Protéine produit par une espèce animale différente de celle utilisée pour la production de primates précédemment immobilisés; la formation du complexe entre le système antigène/anticorps sur le PVDF et un anticorps secondaire dirigé contre les espèces du deuxième type d'anticorps primaires auxquels un système est lie de préférence phosphate alcalin ou peroxydase, ce qui vous permet d'amplifier le signal; détection et/ou quantification des protéines de pointe SARS-CoV-2 et des protéines nucléosides du SARS-CoV-2 par rapport à un échantillon témoin.

3. Kit pour effectuer la méthode telle que définie dans les revendication 1 et 2 comprenant des moyens de détection et/ou de quantification des deux protéines du virus SARS-CoV2: protéine de pointe SARS-CoV-2 et protéine nucléocapside de SARS-CoV-2.

4. Kit selon la revendication 3 comprenant : a) 2 ensembles d'anticorps primaires ant-SARS-CoV-2 Spike Protéine et ant-SARS-CoV-2 Nucléocapside Protéine de deux espèces animales différentes b) système de détection et/ou quantification d'au moins la protéine de la nucléocapside du SARS-CoV-2 à partir d'un complexe protéique SARS-CoV-2 Spike Protéine/ant-SARS-CoV-2 Spike Protéine et SARS-CoV-2 Nucléocapside protéine/ ant-SARS-CoV-2 Nucléocapside protéine constituée d'un anticorps secondaire conjugué avec enzyme pour réaction avec phosphate alcaline ou pour réaction avec peroxydase, dirigée contre l'une des deux espèces d'animaux à partir de laquelle les anticorps primaires sont origine.

5. Kit selon les revendication 3 et 4, dans lequel les deux ensembles d'anticorps ant-SARS-CoV-2 Spike Protéine et ant-SARS-CoV-2 Nucléocapside Protéine sont immobilisés sur un support solide de PVDF et l'autre séché *I* gel pour être soluble au moment de l'utilisation.

6. Kit selon l'une des revendications de 3 à 5, y compris au moins une solution tampon, une solution de lyse et un système de détection.

7. Kit selon la revendication 6 dans laquelle lad souillée solution tampon, la solution lyse et le système de détection sont fournis dans plusieurs puits regroupés en un seul conteneur.

8. Utilisez le kit selon l'une des réclamations de 3 à 7 pour exécuter la méthode telle que définie par les réclamations 1 et 2.

9. Utiliser selon la revendication 8, y compris les étapes suivantes: l'immersion de l'échantillon biologique dans une solution de lyse pour obtenir une solution dans laquelle les protéines de pointe SARS-CoV-2 et les protéines nucléosides du SARS-CoV-2 sont libérées, si elles sont présentes dans l'échantillon; formation de complexes primaires d'antigène/anticorps en contactant la solution de lyse avec la première série d'anticorps primaires ant-SARS-CoV-2 Spike Protéine et ant-SARS-CoV-2 Nucléocapside Protéine, préemptive ment immobilisé sur un membrane PVDF; formation d'un complexe d'anticorps/anticorps avec la deuxième série d'anticorps primaires dans la protéine de pointe ant-SARS-CoV-2 et la protéine nucléocapside ant-SARS-CoV-2 et enfin l'immersion dans une solution comprenant un anticorps secondaire, de la même espèce que les anticorps primaires en solution, équipes d'un système d'enzymes révélatrices; l'immersion de ces complexes immobilisés PVDF dans une solution comprenant un substrat pour la détection de l'anticorps secondaire ; détecter et/ou quantifier la protéine Spike SARS-CoV-2 et la protéine nucléocapside SARS-Co V-2 contre un échantillon témoin.

10. Kit selon la revendication 1 Il est utilisé pour vérifier la contamination par le SARS-COV-2 dès les surfaces et/ou dès les circuits de climatisation et des systèmes de ventilation des bâtiments publics, privés notamment les hôpitaux, mais pas seulement, et les moyenne de transports publics, privés, en particulier avions train navires bus.
